Europäisches Patentamt

European Patent Office

Office européen des brevets

(11) Publication number: **0 334 391 B1**

(12) **EUROPEAN PATENT SPECIFICATION**

(45) Date of publication of patent specification: **12.05.93**

(51) Int. Cl.5: **C12N 15/03**, C12P 13/06, C12N 1/21

(21) Application number: **89108164.8**

(22) Date of filing: **16.02.84**

(60) Publication number of the earlier application in accordance with Art.76 EPC: **0 136 359**

(54) **Process for preparing L-isoleucine.**

(30) Priority: **17.02.83 JP 25397/83**
**17.02.83 JP 25398/83**
**28.05.83 JP 94392/83**
**29.07.83 JP 138775/83**
**04.08.83 JP 142804/83**
**24.09.83 JP 176758/83**

(43) Date of publication of application:
**27.09.89 Bulletin 89/39**

(45) Publication of the grant of the patent:
**12.05.93 Bulletin 93/19**

(84) Designated Contracting States:
**AT BE CH DE FR GB LI NL SE**

(56) References cited:
**EP-A- 0 071 023**

(73) Proprietor: **KYOWA HAKKO KOGYO CO., LTD.**
**6-1, Ohtemachi 1-chome**
**Chiyoda-ku Tokyo 100(JP)**

(72) Inventor: **Oka, Tetsuo**
**2360-17, Naramachi Midori-ku**
**Yokohama-shi Kanagawa 227(JP)**
Inventor: **Katsumata, Ryoichi**
**1380, Yamazaki-machi**
**Machida-shi, Tokyo(JP)**
Inventor: **Ozaki, Akio**
**6-6, Asahimachi 3-chome**
**Machida-shi Tokyo 194(JP)**
Inventor: **Mizukami, Toru**
**14-10, Asahimachi 2-chome**
**Machida-shi Tokyo 194(JP)**
Inventor: **Yokoi, Haruhiko**
**6-6, Asahimachi 3-chome**
**Machida-shi Tokyo 194(JP)**
Inventor: **Hara, Masako**
**40-11, Sagamigaoka 5-chome**
**Zama-shi Kanagawa 228(JP)**

(74) Representative: **Vossius & Partner**
**Siebertstrasse 4 P.O. Box 86 07 67**
**W-8000 München 86 (DE)**

EP 0 334 391 B1

Rank Xerox (UK) Business Services
(3.10/3.6/3.3.1)

**Description**

This invention relates to a process for producing L–isoleucine by a novel method for the expression of a gene. More specifically, the present invention relates to a process for producing L–isoleucine by transforming a host microorganism belonging to the genus Corynebacterium or Brevibacterium with a recombinant vector comprising a DNA fragment containing the threonine operon of Escherichia coli, culturing the transformant in a nutrient medium, accumulating the L–isoleucine in the culture medium and recovering it therefrom.

For the direct production of amino acids by fermentation, methods using wild–type strains, or mutant strains such as auxotrophic strains and amino acid analog–resistant strains, of the bacteria belonging to the genus Corynebacterium, Brevibacterium, Serratia, and the like are known.

For example, the production of histidine using a strain resistant to histidine analog (Japanese Patent Publication No. 8596/81), production of tryptophan using a strain resistant to tryptophan analog (Japanese Patent Publication Nos. 4505/72 and 19037/76), production of isoleucine using a strain resistant to isoleucine analog (Japanese Patent Publication Nos. 38995/72, 6237/76 and 32070/79), production of phenylalanine using a strain resistant to phenylalanine analog (Japanese Patent Publication No. 10035/81), production of tyrosine using a strain requiring phenylalanine for its growth or being resistant to tyrosine [Agr. Chem. Soc., Japan 50 (1) R79–R87 (1976)], production of arginine using a strain resistant to L–arginine analog [Agr. Biol. Chem., 36, 1675–1684 (1972), Japanese Patent Publication Nos. 37235/79 and 150381/82] and the like are known.

The present invention relates to the production of L–isoleucine using a microorganism belonging to the genus Corynebacterium or Brevibacterium by recombinant DNA technology different from the conventional mutational breeding technology for the purpose of improving the L–isoleucine productivity.

The present inventors have constructed plasmid vectors autonomously replicable in a microorganism belonging to the genus Corynebacterium or Brevibacterium and having selectable markers and adequate cloning sites and have developed a highly efficient transformation system (Japanese Published Unexamined Patent Application Nos. 183799/82, 186492/82 and 186489/82). Further, the present inventors have found that the plasmid vectors are useful for expressing a foreign gene in a host microorganism and increasing the productivity of amino acids by ligating a DNA fragment containing a foreign gene involved in the biosynthesis of amino acids such as glutamic acid and lysine to the plasmid vectors according to the procedures in recombinant DNA technology (Methods in Enzymology 68, Recombinant DNA, edited by Ray Wu, Academic Press 1979, U.S. Patent No. 4,237,224) and transforming Corynebacterium glutamicum L–22 or its derivatives using the transformation methods developed by the present inventors (Japanese Published Unexamined Patent Application No. 126789/83).

The present inventors have found that microorganisms prepared by the above method acquire an increased productivity of isoleucine.

No example of expressing a desired gene and increasing the productivity of L–isoleucine in a host microorganism belonging to the genus Corynebacterium or Brevibacterium by introducing a recombinant DNA containing such desired gene and vector, one of which is foreign to a host microorganism, into such host microorganism has been reported.

The present invention is explained in detail below.

The present invention provides a process for producing L–isoleucine by cultivating in a medium a transformant which is obtained by transforming a microorganism belonging to the genus Corynebacterium or Brevibacterium with a recombinant vector comprising a DNA fragment containing the threonine operon of Escherichia coli, accumulating L–isoleucine in the culture medium and recovering it therefrom.

As a preferable source of the threonine operon from Escherichia coli, the recombinant plasmid pEthrl containing the threonine operon DNA of Escherichia coli K–12 is used.

The vector used in the present invention should autonomously replicate in cells of the host microorganism. Preferably, plasmids isolated from microorganisms belonging to the genus Corynebacterium by the present inventors or derivatives thereof such as pCG1 (Japanese Published Unexamined Patent Application No. 134500/82), pCG2 (Japanese Published Unexamined Patent Application No. 35197/83), pCG4 (Japanese Published Unexamined Patent Application No. 183799/82), pCE51, pCE52 (Japanese Published Unexamined Patent Application No. 126789/83), pCE53 (Japanese Published Unexamined Patent Application No. 25398/83), pCE54, pCG11, pCB100 (Japanese Published Unexamined Patent Application No. 105999/83), and the like are mentioned.

Microorganisms carrying these plasmids have been deposited with the Fermentation Research Institute, Agency of Industrial Science and Technology, and the American Type Culture Collection, U.S.A. under the following accession numbers.

2

| Plasmid | FERM P − | ATCC |
|---------|----------|------|
| pCG1 | 5865 | 31808 |
| pCG2 | 5954 | 31832 |
| pCG4 | 5939 | 31830 |
| pCE54 | − | 39019 |
| pCG11 | − | 39022 |
| pCB101 | − | 39020 |

Plasmids pCE51, 52, 53 and 54 can be introduced from the plasmids mentioned above as follows.

For example, pCE51 is prepared as follows.

pCG1 is isolated from the cultured cells of Corynebacterium glutamicum 225 − 57 (FERM P − 5865, ATCC 31808) by the method described in the specification of Japanese Published Unexamined Patent Application No. 134500/82. pGA22 is isolated from the cultured cells of Escherichia coli harboring the plasmid by a conventional method [An, G. et al.,: J. Bacteriol., 140, 400 (1979)]. Plasmid pCG1 is linearized with restriction endonuclease BglII and a fragment of pGA22 digested with BamHI and containing kanamycin resistance (Km$^R$) gene is ligated to the linearized pCG1 using the same cohesive ends of both plasmids. Isolation of pCE51 from the ligated DNA mixture is achieved by selecting the transformants belonging to the genus Corynebacterium or Brevibacterium and containing Km$^R$ derived from pGA22, and analyzing the plasmid in the transformant.

pCE51 has a molecular weight of about 6 Kb and cleavage sites for HincII, HindIII, SmaI, XhoI and EcoRI and gives Km$^R$ phenotype.

pCE52 and pCE53 are prepared as follows.

Plasmid pCG1 is isolated from the cultured cells of Corynebacterium glutamicum 225 − 57 (FERM P − 5865, ATCC 31808) by the method described in the above application and plasmid pGA22 is isolated from the cultured cells of Escherichia coli harboring the plasmid by a conventional method. pCG1 with a unique BglII site is linearized with restriction enzyme BglII and pGA22 with two BamHI sites are partially digested with BamHI. The cohesive ends of both plasmids are annealed and ligated with T4 phage DNA ligase to make a composite molecule. Selection of the recombinant plasmids from the ligation mixture is carried out by isolating transformants of the genus Corynebacterium or Brevibacterium on the basis of drug − resistances derived from pGA22 and then analyzing the plasmids in the transformants.

pCE52 and pCE53 have a molecular weight of about 10.9 Kb and cleavage sites for EcoRI, SalI, SmaI and XhoI. While pCE52 gives the phenotypes of resistance to chloramphenicol (Cm$^R$) and Km$^R$, pCE53 gives resistance to tetracycline (Tc$^R$), Cm$^R$ and Km$^R$ phenotypes. Since the cleavage site for XhoI is present in the Km$^R$ gene, selection by insertional inactivation (prevention of the expression of a gene by the insertion of a DNA fragment into the gene) is possible.

Transformation with the ligated DNA mixture is carried out using protoplasts of the genus Corynebacterium or Brevibacterium, and the method described in Japanese Published Unexamined Patent Application Nos. 186492/82 and 186489/82.

Among the genes responsible for drug resistance derived from pGA22, those except for the resistance gene which is insertionally inactivated are used for selection. In no DNA adding system, transformants are recovered as a colony regenerated on a hypertonic agar medium containing generally 0.4 − 1.6 $\mu$g/m$\ell$ Tc, 2.5 − 5 $\mu$g/m$\ell$ Cm, 100 − 800 $\mu$g/m$\ell$ Km, 100 − 400 $\mu$g/m$\ell$ Sm or 200 − 1,000 $\mu$g/m$\ell$ Spec which does not allow the reversion to normal cells of the recipient protoplasts which are not treated with the ligation mixture. Alternatively, transformants are regenerated unselectively on a regeneration medium, and the resultant cells are scraped and resuspended, followed by the isolation of those cells grown on an agar medium containing a drug in a concentration wherein the recipient normal cells can not grow, that is, generally 0.5 − 4 $\mu$g/m$\ell$ Tc, 2 − 15 $\mu$g/m$\ell$ Cm, 2 − 25 $\mu$g/m$\ell$ Km, 5 − 50 $\mu$g/m$\ell$ Sm or 50 − 500 $\mu$g/m$\ell$ Spec. Some of the transformants selected by Tc$^R$, Cm$^R$ or Km$^R$ are simultaneously endowed with other drug − resistances derived from plasmid pGA22.

Plasmid DNAs in these transformants can be isolated from cultured cells of the transformants and purified according to the methods described in Japanese Published Unexamined Patent Application Nos. 134500/82 and 186489/82. The structures of the DNAs can be determined by digesting them with various restriction endonucleases and analyzing the DNA fragments by agarose gel electrophoresis. The plasmids isolated from the transformants are pCE51, pCE52 and pCE53. Recovery of plasmids from the strains is carried out according to the methods described in Japanese Published Unexamined Patent Application Nos. 134500/82, 183799/82 and 35197/83. Preparation of a recombinant DNA of a vector DNA with a DNA fragment containing a gene is carried out by conventional in vitro recombinant DNA technology.

In vitro recombinant DNA technology is carried out by cleavage and ligation of a donor DNA containing a desired gene to a vector DNA (refer to Japanese Published Unexamined Patent Application No. 126789/83, USP 4,237,224).

The ligase reaction gives recombinants containing genes other than the desired gene. The desired recombinant DNA can be obtained by directly transforming a microorganism of the genus Corynebacterium or Brevibacterium with the ligated DNA mixture, selecting the transformants having the phenotype derived from the desired gene and isolating the desired recombinant DNA from the cultured cells of the transformants. Instead of cloning the desired gene directly in a microorganism of the genus Corynebacterium or Brevibacterium, the desired gene can be cloned by using another host – vector system such as Escherichia coli. Then, it is recloned in vitro into a vector of the genus Corynebacterium or Brevibacterium to transform these microorganisms and transformants containing the desired recombinant plasmid are selected as mentioned above.

The method of cloning a gene in a host microorganism of Escherichia coli is described in, for example, Methods in Enzymology, 68, Ray Wu (Ed) Academic Press New York (1979).

The following references are helpful for the construction of recombinant DNA:

S.N. Cohen, et al., U.S.P. No. 4,237,224;

Idenshi Sosa Jikkenho, edited by Yasuyuki Takagi, printed by Kodansha Scientific (1980);

Methods in Enzymology 68, Recombinant DNA, edited by Ray Wu, Academic Press, 1979

Japanese Published Unexamined Patent Application No. 126789/83.

Microorganisms belonging to the genus Corynebacterium or Brevibacterium and which are competent for incorporating DNAs may be used as the host microorganisms in the present invention. The following are examples of a suitable host microorganism.

| | Accession Number | |
|---|---|---|
| | FERM P – | ATCC |
| Corynebacterium glutamicum | | 13032 |
| Corynebacterium glutamicum L – 15 | 5946 | 31834 |
| Corynebacterium glutamicum LA – 105 | | |
| Corynebacterium glutamicum K – 38 | 7087 | |
| Corynebacterium glutamicum K – 43 | 7162 | |
| Corynebacterium herculis | | 13868 |
| Corynebacterium herculis L – 103 | 5947 | 31866 |
| Corynebacterium acetoacidophilum | | 13870 |
| Corynebacterium lilium | | 15990 |
| Brevibacterium divaricatum | | 14020 |
| Brevibacterium divaricatum L – 204 | 5948 | 31867 |
| Brevibacterium lactofermentum | | 13869 |
| Brevibacterium lactofermentum L – 312 | 5949 | 31868 |
| Brevibacterium flavum | | 14067 |
| Brevibacterium immariophilium | | 14068 |
| Brevibactrerium thiogenitalis | | 19240 |

Transformation of the host microorganisms with recombinant DNAs is carried out by the following steps:

1) Preparation of protoplasts from cultured cells;

2) Transformation of the protoplasts with a recombinant DNA;

3) Regeneration of the protoplasts to normal cells and selection of a transformant;

These steps are described in detail below.

1. Preparation of protoplasts from cultured cells:

The preparation of protoplasts is carried out by culturing a microorganism under conditions which render it sensitive to lysozyme, a lytic enzyme, and treating the cultured cells with lysozyme in a hypertonic solution to remove the cell wall. In order to render microbial cells sensitive to lysozyme, reagents inhibiting the synthesis of bacterial cell walls are used. For example, microbial cells sensitive to lysozyme are obtained by adding, during the logarithmic growth phase, an amount of penicillin which does not inhibit or sub – inhibits the growth and then continuing culturing for several generations.

4

For culturing, any medium wherein the microorganism can grow may be used. For example, a nutrient medium NB (pH 7.2) consisting of 20 g/ℓ powdered bouillon and 5 g/ℓ yeast extract and a semi − synthetic medium SSM (pH 7.2) consisting of 10 g/ℓ glucose, 4 g/ℓ $NH_4Cl$, 2 g/ℓ urea, 1 g/ℓ yeast extract, 1 g/ℓ $KH_2PO_4$, 3 g/ℓ $K_2HPO_4$, 0.4 g/ℓ $MgCl_2 \cdot 6H_2O$, 10 mg/ℓ $FeSC_4 \cdot 7H_2O$, 0.2 mg/ℓ $MnSO_4 \cdot (4−6)H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6Mo_7O_{24} \cdot 4H_2O$, 30 μg/ℓ biotin and 1 mg/ℓ thiamine hydrochloride are used.

Microbial cells are inoculated in the medium and culturing is carried out with shaking.

The optical density (CD) of the culture medium at 660 nm is monitored with a colorimeter and penicillin, such as penicillin G, is added to the medium at an initial stage of the logarithmic growth phase (OD : 0.1 − 0.4) in a concentration of 0.1 to 2.0 U/mℓ. Culturing is continued to an OD value of 0.3 − 0.5, and then cells are harvested and washed with the SSM medium. The washed cells are resuspended in a suitable hypertonic medium such as PFM medium (pH 7.0 − 8.5) wherein 0.4M sucrose and 0.01M $MgCl_2 \cdot 6H_2O$ are added to 2 fold diluted SSM medium, and RCG medium (pH 7.0 − 8.5) consisting of 5 g/ℓ glucose, 5 g/ℓ casein hydrolysate, 2.5 g/ℓ yeast extract, 3.5 g/ℓ $K_2HPO_4$, 1.5 g/ℓ $KH_2PO_4$, 0.41 g/ℓ $MgCl_2 \cdot 6H_2O$, 10 mg/ℓ $FeSO_4 \cdot 7H_2O$, 2 mg/ℓ $MnSO_4 \cdot (4−6)H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6Mo_7O_{24} \cdot 4H_2O$, 30 μg/ℓ biotin, 2 mg/ℓ thiamine hydrochloride and 135 g/ℓ sodium succinate or RCGP medium which consists of RCG medium and 3% polyvinyl pyrrolidone. To the cell suspension, lysozyme is added to a final concentration of 0.2 to 10 mg/mℓ, and the mixture is allowed to react at a temperature of 30 to 37˚C. Protoplast formation proceeds with time and is monitored with an optical microscope. The period required for the conversion of most cells to protoplasts depends on the concentrations of the penicillin used for the lysozyme − sensitization and the amount of lysozyme used. The period is 3 − 24 hours under the conditions mentioned above.

Since protoplasts formed are destroyed under hypotonic conditions, the extent of the formation of protoplast is determined indirectly from the number of normal cells surviving under hypotonic conditions. Generally, the ratio of surviving normal cells are kept below $10^{-4}$ per lysozyme − treated normal cell.

The protoplasts as prepared above have colony − forming (regenerating) ability on a suitable hypertonic agar medium. As the agar medium, a nutrient medium, a semi − synthetic medium or a synthetic medium containing various amino acids, which contains 0.3 to 0.8M sodium succinate and 0.5 to 6% polyvinyl pyrrolidone with a molecular weight of 10,000 or 40,000 is preferably used. Generally, a semi − synthetic medium RCGP (pH 7.2) wherein 1.4% agar is added to the RCGP agar medium is used. Regeneration is carried out at a temperature of 25 to 35˚C. The cultivation time required for the regeneration of protoplasts depends upon the strain used, and usually in 10 to 14 days colonies can be picked up. The efficiency of the regeneration of protoplasts on the RCGP medium also depends on the strain used, the concentrations of the penicillin added during the cultivation and the concentration of lysozyme used. The efficiency is generally $10^{-2}$ − $10^{-4}$ cells per normal cell treated with lysozyme.

2. Transformation of the protoplasts with a recombinant DNA:

Introduction of a recombinant DNA into the protoplast is carried out by mixing the protoplast and the DNA in a hypertonic solution which protects the protoplast and by adding to the mixture polyethyleneglycol (PEG, average molecular weight: 1,540 − 6,000) or polyvinylalcohol (PVA, degree of polymerization: 500 − 1,500) and a divalent metal cation which stimulates the uptake of DNA. As a stabilizing agent for the hypertonic conditions, those generally used to protect protoplasts of other microorganisms such as sucrose and sodium succinate are also employed. PEG and PVA can be used at a final concentration of 5 to 60% adn 1 to 20%, respectively. Divalent metal cations such as $Ca^{++}$, $Mg^{++}$, $Mn^{++}$, $Ba^{++}$, and $Sr^{++}$ are effectively used alone or in combination at a final concentration of 1 to 100 mM. Transformation is carried out satisfactorily at 0 to 25˚C.

3. Regeneration of the protoplasts to normal cells and selection of a transformant:

Regeneration of the protoplast transformed with a recombinant DNA is carried out in the same way as mentioned above by spreading the protoplast on a hypertonic agar medium such as RCGP medium containing sodium succinate and polyvinyl pyrrolidone and incubating at a temperature wherein normal cells can grow, generally 25 to 35˚C. Transformants are obtained by selecting the phenotypes derived from donor DNAs. The selection by characteristic phenotype endowed may be carried out simultaneously with regeneration on a hypertonic agar medium or may be carried out on a hypotonic agar medium after non − selective reversion to normal cells on a hypertonic agar medium.

In the case of using the lysozyme‑sensitive strains described as the preferred host microorganisms, transformation may be carried out by the steps described in (1) to (3) except that the cultured cells are directly treated with lysozyme without prior treatment with penicillin in step (1). In that case, transformants are obtained at an efficiency of $10^{-2}$ to $10^{-4}$ per regenerated cell.

The following strains are the examples of the transformants obtained by the present invention.

Isoleucine‑producing strains

Corynebacterium glutamicum K41, FERM P‑7161

Brevibacterium flavum K42, FERM BP‑355

The phenotypic expression of the recombinant DNA is carried out by growing the transformant in a conventional nutrient medium. Appropriate reagents may be added to the medium according to the phenotypes expected from the gene DNA or vector DNA on the recombinant DNA.

The thus obtained transformant is cultured in a conventional manner used in the production of amino acids by fermentation. That is, the transformant is cultured in a conventional medium containing carbon sources, nitrogen sources, inorganic materials, amino acids, vitamins, etc. under aerobic conditions, with adjustment of temperature and pH. Amino acids, thus accumulated in the medium are recovered.

As the carbon source, various carbohydrates such as glucose, glycerol, fructose, sucrose, maltose, mannose, starch, starch hydrolyzate and molasses, polyalcohols and various organic acids such as pyruvic acid, fumaric acid, lactic acid and acetic acid may be used. According to the assimilability of the microorganism strain used, hydrocarbon and alcohols are employed. Blackstrap molasses is most prefer‑ably used.

As the nitrogen source, ammonia, various inorganic or organic ammonium salts such as ammonium chloride, ammonium sulfate, ammonium carbonate and ammonium acetate, urea, and nitrogenous organic substances such as peptone, NZ‑amine, meat extract, yeast extract, corn steep liquor, casein hydrolyzate, fish meal or its digested product, defatted soybean or its digested product and chrysalis hydrolyzate are appropriate.

As the inorganic materials, potassium dihydrogenphosphate, dipotassium hydrogenphosphate, ammo‑nium sulfate, ammonium chloride, magnesium sulfate, sodium chloride, ferrous sulfate, manganese sulfate and calcium carbonate may be used. Vitamines and amino acids required for the growth of microorganisms may not be added, provided that they are supplied with other components mentioned above.

Culturing is carried out under aerobic conditions with shaking or aeration‑agitation. Culturing tem‑perature is preferably 20 to 40°C. The pH of the medium during culturing is maintained around neutral. Culturing is continued until a considerable amount of an amino acid is accumulated, generally for 1 to 5 days.

After completion of the culturing, cells are removed and the amino acid is recovered from the culture liquor by conventional manners such as treatment with active carbon or ion exchange resin.

In spite of the high similarity in microbiological characteristics, so called glutamic acid‑producing microorganisms which produce glutamic acid in large amounts are classified into various species and even into different genera such as Corynebacterium and Brevibacterium, which is probably because of their industrial importance. However, it has been pointed out that these microorganisms should belong to one species because of nearly the same composition of amino acids in the cell wall and the base composition of DNAs. Recently, it has been reported that these microorganisms have at least 70 to 80% homology in DNA‑DNA hybridization, indicating that these microorganisms are closely related. See, e.g., Komatsu, Y.: Report of the Fermentation Research Institute, No. 55, 1 (1980), and Suzuki, K., Kaneko, T., and Komagata, K.: Int. J. Syst. Bacteriol., 31, 131 (1981).

In consideration of the facts mentioned above, it is readily assumed that the usefulness of the present invention is applicable to all the glutamic acid‑producing microorganisms. In order to stably maintain recombinant DNA molecules and express the DNA in these species, slight differences of such properties of the host microorganisms as homology in the DNA are negligible and it is sufficient for host microorganisms to allow the autonomous replication of plasmids and expression of genes on them. That these microorgan‑isms have such abilities is apparent from the facts that plasmid pCG4 which was isolated from Corynebac‑terium glutamicum 235‑250 (Japanese Published Unexamined Patent Application No. 183799/82) and having an streptomycin and/or spectinomycin resistant gene could replicate in the glutamic acid‑producing microorganisms such as those belonging to the genus Corynebacterium or Brevibacterium and that the gene responsible for the resistance could be expressed (Japanese Published Unexamined Patent Applica‑tion No. 136492/82). Further, as described in the production of histidine of the parent application of the present application which matured into European patent No. 136359, it is apparent that the gene expressed in Corynebacterium glutamicum is expressible in broad host microorganisms of the genera Corynebac‑

6

terium, Brevibacterium and the like. Therefore, all the glutamic acid – producing microorganisms including those belonging to the genus Corynebacterium or Brevibacterium as well as the species Corynebacterium glutamicum are competent as the host microorganism of the present invention.

For the strains appearing in the present specification, the accession numbers, deposition date and transfer date of the deposits under the Budapest Treaty of the International Recognition of the Deposit of Microorganisms for the purposes of Patent Procedure are as follows.

| | ATCC | FERM P (BP) | Deposition date (Transfer date) Year/Month/Day |
|---|---|---|---|
| Corynebacterium glutamicum L-15 | 31834 | | 1981. 3. 9 ( ) |
| Corynebacterium herculis L-103 | 31366 | | 1981. 4. 3 ( ) |
| Brevibacterium divaricatum L-204 | 31367 | | 1981. 4. 3 ( ) |
| Brevibacterium lactofermentum L-312 | 31368 | | 1981. 4. 3 ( ) |
| Corynebacterium glutamicum LA103/pCB54 | 39019 | | 1981.12.11 |
| Corynebacterium glutamicum LA103/pCB101 | 39020 | | 1981.12.11 |
| Corynebacterium glutamicum LA103/pEthr1 | 39021 | | 1981.12.11 |
| Corynebacterium glutamicum LA103/pCG11 | 39022 | | 1981.12.11 |
| Corynebacterium glutamicum K17 | 39032 | | 1981.12.21 |
| Corynebacterium glutamicum K18 | 39033 | | 1981.12.21 |
| Corynebacterium glutamicum K19 | 39034 | | 1981.12.21 |
| Corynebacterium glutamicum K20 | 39035 | | 1981.12.21 |
| Corynebacterium glutamicum K31 | 39280 | | 1983. 1.28 |
| Corynebacterium glutamicum K32 | 39281 | | 1983. 1.28 |
| Corynebacterium herculis K33 | 39282 | | 1983. 1.28 |
| Brevibacterium flavum K34 | 39283 | | 1983. 1.28 |
| Brevibacterium lactofermentum K35 | 39294 | | 1983. 1.28 |

8

| | | |
|---|---|---|
| Corynebacterium glutamicum K37 | 39285 | 1983. 1.31 |
| Corynebacterium glutamicum K36 | 6908 ( 451) | 1983. 2. 3 (1984. 1.19) |
| Corynebacterium glutamicum K33 | 6909 ( 452) | 1983. 2. 3 (1984. 1.19) |
| Corynebacterium glutamicum C156 | 6910 ( 453) | 1983. 2. 3 (1984. 1.19) |
| Corynebacterium glutamicum K38 | 7087 ( 454) | 1983. 5.19 (1984. 1.19) |
| Corynebacterium glutamicum K39 | 7088 ( 459) | 1983. 5.19 (1984. 1.21) |
| Corynebacterium glutamicum K40 | 7160 ( 455) | 1983. 7.21 (1984. 1.19) |
| Corynebacterium glutamicum K41 | 7161 ( 456) | 1983. 7.21 (1984. 1.19) |
| Corynebacterium glutamicum K43 | 7162 ( 457) | 1983. 7.21 (1984. 1.19) |
| Corynebacterium glutamicum K44 | 7163 ( 458) | 1983. 7.21 (1984. 1.19) |
| Corynebacterium glutamicum K45 | 7164 ( 460) | 1983. 7.21 (1984. 1.21) |
| Brevibacterium *flavum* K42 | ( 355) | 1983. 9.12 |
| Corynebacterium glutamicum K46 | ( 356) | 1983. 9.12 |
| Brevibacterium *flavum* K48 | ( 357) | 1983. 9.12 |
| Corynebacterium herculis K47 | ( 367) | 1983. 9.21 |
| Corynebacterium glutamicum K49 | ( 464) | 1984. 1.25 |

Fig. 1 illustrates the cleavage pattern with restriction endonucleases of pGH2.

Fig. 2 illustrates the process for construction of and the cleavage map for restriction endonucleases of pEthrI wherein "Bgl II/Bam HI" with a broken line indicates a recombination site at the same cohesive ends formed by cleavage with both restriction endonucleases. The restriction endonucleases used in the preparation of the cleavage map are PstI, EcoRI and XhoI. Molecular weight of the plasmid is indicated as Kilobase pairs (Kb).

Example 1

Preparation of DNA of plasmid pCG11:

pCG11 used as a vector plasmid was prepared from Corynebacterium glutamicum LA 103/pCG11, ATCC 39022 which is a derivative of Corynebacterium glutamicum L − 22 and harbors pCG11 as follows.

The strain was grown with shaking at 30°C in 400 mℓ of NB medium to an OD value of about 0.7. Cells were harvested and washed with TES buffer. The cells were suspended in 10 mℓ of the aforementioned lysozyme solution and allowed to react at 37°C for 2 hours. Then 2.4 mℓ of 5M NaCl, 0.6 mℓ of 0.5M EDTA (pH 8.5) and 4.4 mℓ of a solution consisting of 4% sodium lauryl sulfate and 0.7M NaCl were added successively. The mixture was stirred slowly and allowed to stand on an ice water bath for 15 hours.

The whole lysate was centrifuged at 4°C under 69,400 x g for 60 minutes. The supernatant fluid was recovered and 10% (by weight) polyethyleneglycol (PEG) 6,000 (product of Nakarai Kagaku Yakuhin Co.) was added. The mixture was stirred slowly to dissolve completely and then kept on an ice water bath. After 10 hours, the mixture was subjected to centrifugation at 1,500 x g for 10 minutes to recover a pellet. The pellet was redissolved gently in 5 mℓ of TES buffer and 2.0 mℓ of 1.5 mg/mℓ ethidium bromide was added. Then, cesium chloride was added to adjust the density of the mixture to 1.580. The solution was centrifuged at 18°C at 105,000 x g for 48 hours. After the density gradient centrifugation, a covalently − closed circular DNA was detected under UV irradiation as a high density band located in the lower part of the centrifugation tube. The band was taken out from the side of the tube with an injection needle to obtain a fraction containing pCG11 DNA. To remove ethidium bromide, the fraction was treated five times with an equal amount of cesium chloride saturated isopropyl alcohol solution consisting of 90% by volume isopropyl alcohol and 10% TES buffer solution. Then, the residue was dialysed against TES buffer solution to obtain pCG11 plasmid DNA.

Example 2

(1) Cloning of a DNA fragment containing Escherichia coli threonine operon:

Cloning was carried out using a host − vector system of Escherichia coli. Plasmid pGA22, used as a vector, was isolated from cultured cells of a derivative of Escherichia coli K12 carrying the present plasmid according to the method of An [An, G. et al.: J. Bacteriol., 140, 400 (1979)]. The chromosomal DNA used as a donor DNA was isolated from cultured cells of Escherichia coli K12 Hfr (ATCC 23740) by the phenol − extraction method of Smith [Smith, M.G.: Methods in Enzymology, 12, part A, 545 (1967)].

Then, 0.4 unit of HindIII (16 units/μℓ) was added to 60 μℓ of a HindIII reaction solution (pH 7.5) consisting of 10 mM Tris, 7 mM MgCl$_2$ and 60 mM NaCl (the same HindIII reaction solution was used hereinafter) and containing 4 μg of pGA22 plasmid DNA. The mixture was allowed to react at 37°C for 30 minutes and heated at 65°C for 10 minutes to stop the reaction. pGA22, which has two HindIII cleavage sites, was digested with HindIII under the same conditions and subjected to agarose gel electrophoresis. It was confirmed that one of the two HindIII cleavage sites present in pGA22 was cleaved. Separately, 4 units of HindIII was added to 140 μℓ of the HindIII reaction solution containing 8 μg of the chromosomal DNA. The mixture was allowed to react at 37°C for 60 minutes and heated at 65°C for 10 minutes to stop the reaction. Then, 40 μℓ of the T4 ligase buffer solution II, 40 μℓ of 5 mM ATP, 0.3 μℓ of T4 ligase and 120 μℓ of H$_2$O were added to a mixture of the above digests and reaction was carried out at 12°C for 16 hours. The reaction mixture was extracted twice with 400 μℓ of phenol saturated with TES buffer solution and subjected to dialysis against TES buffer solution to remove phenol.

The ligase reaction mixture was used to transform Escherichia coli K − 12, GT − 3 (J. Bacteriol. 117, 133, 1974) which is a mutant defective in three aspartate kinases and requiring homoserine and diaminopimelic acid. Competent cells of the GT − 3 strain were prepared according to the method of Dagert, M. et al., Gene, 6, 23 (1979). That is, the strain was inoculated in 50 mℓ of L − medium (pH 7.2) consisting of 10 g/ℓ Bacto − trypton, 5 g/ℓ yeast extract, 1 g/ℓ glucose and 5 g/ℓ sodium chloride and containing 100 μg/mℓ diaminopimelic acid and cultured at 37°C to an optical density (OD) valve at 660 nm of 0.5. The culture was cooled with ice water for 10 minutes and cells were recovered by centrifugation. The cells were suspended in 20 mℓ of cooled 0.1M calcium chloride. The suspension was allowed to stand at 0°C for 20 minutes and then centrifuged to recover the cells. The cells were suspended in 0.5 mℓ of 0.1M calcium chloride and allowed to stand at 0°C for 18 hours. Then, 200 μℓ of the ligase reaction mixture mentioned above was added to 400 μℓ of the cell suspension treated with calcium chloride. The mixture was allowed to stand at 0°C for 10 minutes and then heated at 37°C for 5 minutes. Thereafter, 9 mℓ of the L − medium was added and the mixture was incubated with shaking at 37°C for 2 hours.

Cells were recovered by centrifugation and washed with a physiological saline solution twice. The cells were spread on M9 minimal agar medium (pH 7.2) consisting of 2 g/ℓ glucose, 1 g/ℓ NH₄Cl, 6 g/ℓ Na₂HPO₄, 3 g/ℓ KH₂PO₄, 0.1 g/ℓ MgSO₄·7H₂O, 15 mg/ℓ CaCl₂·2H₂O, 4 mg/ℓ thiamine hydrochloride and 15 g/ℓ agar and containing 12.5 μg/mℓ kanamycin (the same M9 minimal agar medium was used hereinafter). Culturing was carried out at 37°C for 3 days. Only one colony was formed and the cells from this colony could also grow on an agar medium containing 25 μg/mℓ ampicillin, 25 μg/mℓ chloramphenicol or 25 μg/mℓ kanamycin, which is a selection marker of pGA22.

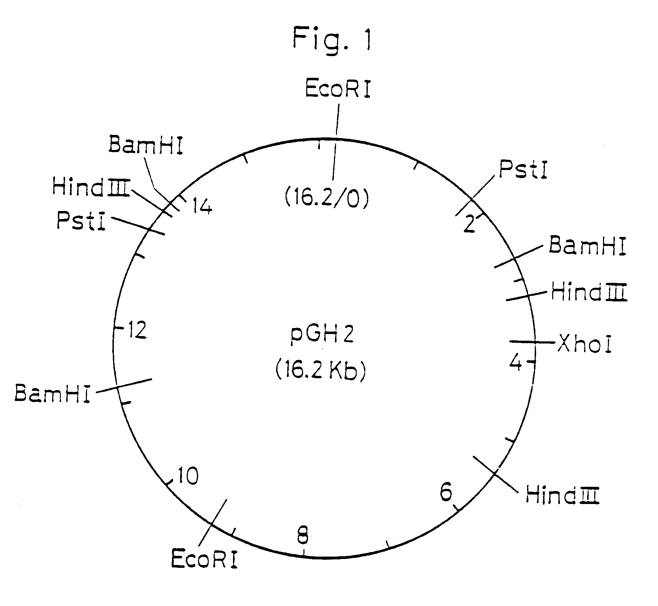

A plasmid DNA was isolated from cultured cells of the transformant by the same method as in the isolation of pGA22. The plasmid DNA was digested with restriction endonucleases and analyzed by agarose gel electrophoresis. The plasmid DNA had the structure illustrated as pGH2 in Fig. 1. Since the DNA fragment inserted in pGA22 had the same cleavage sites for restriction endonucleases as the cloned DNA fragment containing Escherichia coli operon (Cossart, P. et al.: Molec. Gen. Genet., 175, 39, 1979), it is confirmed that pGH2 had the threonine operon.

(2) In vitro recombination of pCG11 and pGH2

2 units of BglII (6 units/μℓ) was added to 100 μℓ of the BglII reaction buffer solution containing 2 μg of pCG11 plasmid DNA obtained by the same method as in Example 1. The mixture was allowed to react at 37°C for 60 minutes. Separately, 2 units of BamHI (6 units/μℓ) was added to 100 μℓ of BamHI reaction buffer solution containing 2 μg of pGH2 plasmid DNA. The mixture was allowed to react at 37°C for 60 minutes. Both digests were heated at 65°C for 10 minutes, and mixed. Then, 40 μℓ of T4 ligase buffer solution II, 40 μℓ of 5 mM ATP, 0.2 μℓ of T4 ligase and 120 μℓ of H₂O were added to the whole mixture of both the digests. Reaction was carried out at 12°C for 16 hours. The reaction mixture was extracted twice with 400 μℓ of phenol saturated with TES buffer solution and subjected to dialysis against TES buffer solution to remove phenol.

(3) Recovery of plasmid pEthrl

Protoplasts of Corynebacterium glutamicum LA201 which requires homoserine and leucine were used for transformation. A seed culture of Corynebacterium glutamicum LA201 was inoculated in NB medium and cultured with shaking at 30°C. Cultured cells were collected at an OD value of 0.6 and suspended in an RCGP medium (pH 7.6) containing 1 mg/mℓ lysozyme at a concentration of about 10⁹ cells/mℓ. The suspension was put into an L−tube and allowed to react with gentle shaking at 30°C for 5 hours to make protoplasts.

Then, 0.5 mℓ of the protoplast suspension was put into a small tube and centrifuged at 2,500 x g for 5 minutes to obtain pellets. The pellets were resuspended in 1 mℓ of a TSMC buffer solution, and subjected to centrifugation and washing. The protoplasts were resuspended in 0.1 mℓ of the TSMC buffer solution. Then, 100 μℓ of a mixture of a two−fold concentrated TSMC buffer solution and the above−described DNA mixture treated with ligase (1:1) was added to the suspension and 0.8 mℓ of a TSMC buffer solution containing 20% PEG 6,000 was added. After 3 minutes, 2 mℓ of the RCGP medium (pH 7.2) was added and the mixture was centrifuged at 2,500 x g for 5 minutes. The supernatant fluid was removed and the precipitated protoplasts were suspended in 1 mℓ of the RCGP medium. The suspension was slowly shaken at 30°C for 2 hours. Then, 0.1 mℓ of the suspension was spread on RCGP agar medium containing 400 μg/mℓ kanamycin, and culturing was carried out at 30°C for 6 days.

Kanamycin−resistant transformants grown over the whole surface of the agar medium were scraped, washed with physiological saline solution and subjected to centrifugation. The cells were spread on a minimal agar medium M1 containing 50 μg/mℓ leucine. Culturing was carried out at 30°C for 3 days. Among colonies developed, those able to grow on the NB agar medium containiing 20 μg/mℓ kanamycin and 100 μg/mℓ spectinomycin were obtained.

Three strains selected at random were grown in 400 mℓ of NB medium to an OD value of about 0.8. Cells were harvested and the plasmids were isolated from the cells by ethidium bromide−cesium chloride density gradient centrifugation described in Example 1 whereby 40 to 55 μg of plasmid DNA were recovered from each strain.

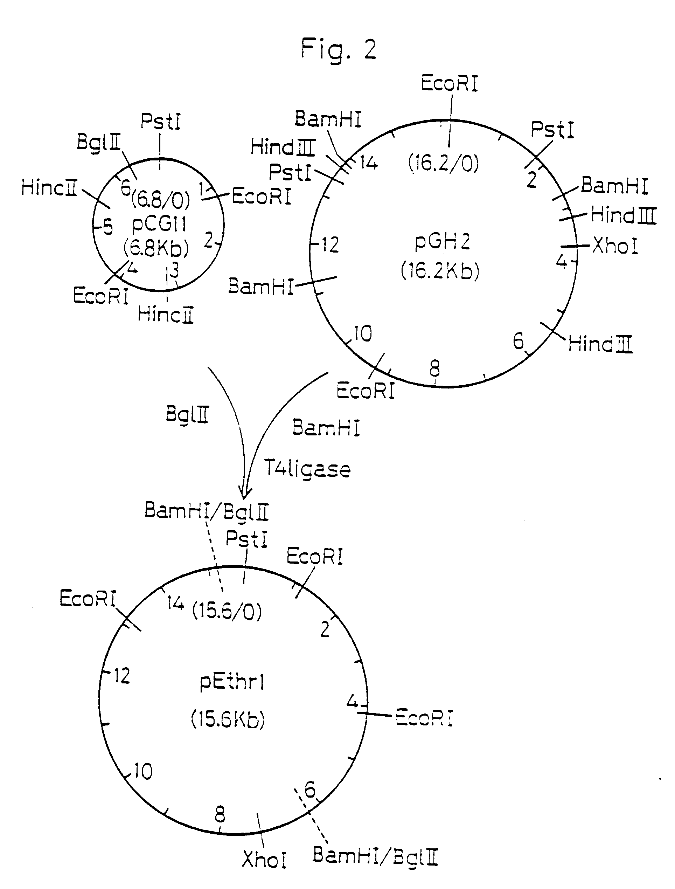

These plasmid DNAs were digested with restriction endonucleases and analyzed by agarose gel electrophoresis to determine the molecular weights and cleavage sites for PstI, EcoRI and XhoI. The plasmid obtained from one strain is named pEthr1 and the structure is illustrated in Fig. 2. It was confirmed that pEthr1 has the structure wherein a BamHI fragment containing pGH2 threonine operon is inserted into pCG11 at its BglII site. One of the remaining strains has the same plasmid as pEthr1 and the other has a plasmid wherein the BamHI fragment containing pGH2 threonine operon is combined at the opposite orientation.

Corynebacterium glutamicum LA201 strain was again transformed with these plasmid DNAs as mentioned above. As a result, strains which do not require homoserine were obtained at high frequency, about $10^{-3}$ cell/regenerated cell. All of them are endowed with the phenotypes of the resistance to kanamycin and spectinomycin and have the same plasmid as the donor plasmid characterized by the cleavage pattern for various restriction endonucleases.

(4) Production of L − isoleucine by the pEthr1 − carrying strain:

The protoplasts of Corynebacterium glutamicum K40 and Brevibacterium flavum ATCC 14067 were transformed with pEthr1. Corynebacterium glutamicum K40 (FERM P − 7160, FERM BP − 455) and Brevibacterium flavum ATCC 14067 were cultured with shaking in NB medium at 30°C for 16 hours, and 0.1 mℓ of the seed culture was inoculated into 10 mℓ of SSM medium in an L − tube. Culturing was carried out at 30°C in a Monod − type culture bath, and penicillin G was added at an OD value of 0.15 to a concentration of 0.5 unit/mℓ. Culturing was continued to an OD value of about 0.6. Cells were harvested and suspended in 2 mℓ of RCGP medium (pH 7.6) containing 1 mg/mℓ lysozyme. The suspension was put in an L − tube and stirred slowly at 30°C for 14 hours to obtain protoplasts.

Then, 1 mℓ of the protoplast suspension was put in a small test tube and centrifuged at 2,500 x g for 15 minutes. The protoplasts were resuspended in 1 mℓ of TSMC buffer and centrifuged at 2,500 x g. The washed protoplasts were resuspended in 0.1 mℓ of TSMC buffer solution. One hundred microliter or a mixture (1:1 by volume) of a two − fold concentrated TSMC buffer and the pEthr1 DNA solution isolated above was added to the protoplast suspension. Transformation was carried out using PEG 6,000 by the same method described in Example 1 for expression of the desired gene. Then, 0.1 mℓ of the mixture was spread on RCGP agar medium contaning 400 µg/mℓ spectinomycin and incubated at 30°C for 10 days. From the colonies developed, the transformants which grow on NB agar medium containing 100 µg/mℓ spectinomycin and 20 µg/mℓ kanamycin were obtained. The strains resistant to spectinomycin and kanamycin were cultured with shaking in 400 mℓ of SSM medium, and penicillin G was added at an OD value of 0.15 to a concentration of 0.5 unit/mℓ. Culturing was continued to an OD value of 0.65, and cells were harvested. From the cells, plasmids were isolated by the same method as the isolation method of pCG11 in Example 1. These plasmids were digested with restriction endonucleases and analyzed by agarose gel electrophoresis. The analysis showed that some of the plasmids have the same physical structure as pEthr1 characterized by the cleavage pattern for various restriction endonucleases. Such transformants are Corynebacterium glutamicum K41 (FERM P − 7161, FERM BP − 456) and Brevibacterium flavum K42 (FERM BP − 355).

Corynebacterium glutamicum K40, Brevibacterium flavum ATCC 14067 and their pEthr1 − carrying strains were tested for L − isoleucine production as follows. The strain was cultured with shaking in NB medium at 30°C for 16 hours and 0.5 mℓ of the seed culture was inoculated into a production medium adjusted to pH 7.2 consisting of 100 g/ℓ glucose, 20 g/ℓ $(NH_4)_2SO_4$, 0.5 g/ℓ $KH_2PO_4$, 0.5 g/ℓ $K_2HPO_4$, 1 g/ℓ $MgSO_4 \cdot 7H_2O$, 10 mg/ℓ $FeSO_4 \cdot 7H_2O$, 10 mg/ℓ $MnSO_4 \cdot (4−6)H_2O$, 100 µg/ℓ biotin and 30 g/ℓ $CaCO_3$ in a test tube. Culturing was carried out with shaking at 30°C for 72 hours. The culture filtrate was subjected to paper chromatography and color reaction with ninhydrin. The amount of L − isoleucine formed was determined colorimetrically. The results are shown in Table I.

## Table I

| Strain | Amount of L-isoleucine (mg/mℓ) |
|---|---|
| Corynebacterium glutamicum K40 | 1.2 |
| Corynebacterium glutamicum K41 | 2.7 |
| Brevibacterium flavum ATCC 14067 | 0 |
| Brevibacterium flavum K42 | 0.8 |

**EP 0 334 391 B1**

**Claims**

1. A process for producing L−isoleucine which comprises culturing in a medium a microorganism obtainable by transforming a host microorganism belonging to the genus Corynebacterium or Brevibacterium with a recombinant vector comprising a DNA fragment containing the threonine operon of Escherichia coli, accumulating L−isoleucine in the culture medium and recovering L−isoleucine therefrom.

2. The process according to claim 1, wherein the recombinant DNA is plasmid pEthr1 (FERM BP−456 or FERM BP−355).

3. The process according to claim 1, wherein the microorganism is Corynebacterium glutamicum K41, FERM BP−456 or Brevibacterium flavum K42, FERM BP−355.

4. A biologically pure culture of Corynebacterium glutamicum K41, FERM BP−456 or Brevibacterium flavum K42, FERM BP−355.


**Patentansprüche**

1. Verfahren zur Herstellung von L−Isoleucin, dadurch gekennzeichnet, daß in einem Medium ein Mikroorganismus kultiviert wird, der durch das Transformieren eines zu der Gattung Corynebacterium oder Brevibacterium gehörenden Wirts−Mikroorganismus mit einem rekombinanten Vektor erhältlich ist, wobei der Vektor ein das Threonin−Operon von Escherichia coli enthaltendes DNA−Fragment umfaßt, L−Isoleucin in dem Kulturmedium akkumuliert wird und L−Isoleucin daraus gewonnen wird.

2. Verfahren nach Anspruch 1, wobei die rekombinante DNA das Plasmid pEthr1 (FERM BP−456 oder FERM BP−355) ist.

3. Verfahren nach Anspruch 1, wobei der Mikroorganismus Corynebacterium glutamicum K41, FERM BP−456 oder Brevibacterium flavum K42, FFRM BP−355 ist.

4. Biologisch reine Kultur von Corynebacterium glutamicum K41, FERM BP−456 oder Brevibacterium flavum K42, FERM BP−355.


**Revendications**

1. Procédé de production de la L−isoleucine, caractérisé en ce que l'on cultive dans un milieu un microorganisme que l'on peut obtenir par la transformation d'un microorganisme hôte appartenant au genre Corynebacterium ou Brevibacterium avec un vecteur recombinant comprenant un fragment d'ADN contenant l'opéron de la thréonine d'Escherichia coli qu'on accumule la L−isoleucine dans la culture et on récupère la L−isoleucine de cette culture.

2. Procédé suivant la revendication 1, caractérisé en ce que l'ADN recombinant est le plasmide pEthrl (FERM BP−456 ou FERM BP−355).

3. Procédé suivant la revendication 1, caractérisé en ce que le microorganisme est Corynebacterium glutamicum K41, FERM BP−456 ou Brevibacterium flavum K42, FERM BP−355.

4. Culture biologiquement pure de Corynebacterium glutamicum K41, FERM BP−456 ou Brevibacterium flavum K42, FERM BP−355.

13

Fig. 1

## Fig. 2